# EUROPÄISCHE PATENTSCHRIFT

(11) **EP 1 316 546 B1**
(45) Veröffentlichungstag und Bekanntmachung des Hinweises auf die Patenterteilung: **13.04.2005**
(21) Anmeldenummer: 02025668.1
(22) Anmeldetag: 20.11.2002
(51) Int. Cl.: C07C 253/30

(54) **Verfahren zur Herstellung von Beta-Ketonitrilen**
Process for the preparation of beta-ketonitriles
Procédé de préparation de beta-cétonitriles

(30) Priorität: 03.12.2001 DE 10159329
(43) Veröffentlichungstag der Anmeldung: 04.06.2003
(73) Patentinhaber: Symrise GmbH & Co. KG, 37603 Holzminden (DE)
(72) Erfinder: Koch, Oskar, Dr., 37079 Göttingen (DE)
(74) Vertreter: Eisenführ, Speiser & Partner

(56) Entgegenhaltungen:
- DE-A- 2 302 273
- EDWIN H. KROEKER ET AL.: "The Preapration of Ethyl alpha-Carbethoxy-alpha'-isobutyryl-beta-ph enylglutarate. Observations on the Retrogression of the Michael Reaction" JOURNAL OF THE AMERICAN CHEMICAL SOCIETY., Bd. 56, Nr. 5, 5. Mai 1934 (1934-05-05), Seiten 1171-1173, XP002234122 AMERICAN CHEMICAL SOCIETY, WASHINGTON, DC., US ISSN: 0002-7863

## Beschreibung

Die vorliegende Erfindung betrifft ein Verfahren zur Herstellung von β-Ketonitrilen.

β-Ketonitrile sind wichtige Zwischenprodukte zur Herstellung von pharmazeutischen (WO-A 99/23091) und agrochemischen Wirkstoffen (EP-A 496 630).

In der EP-A 220 220 wird die Herstellung durch Kondensation von Carbonsäureestern in einem Überschuss Acetonitril unter Verwendung von Natriummethylat beschrieben. Dabei wird das bei der Reaktion entstehende Methanol zusammen mit Acetonitril kontinuierlich abdestilliert, woraus eine Ausbeute von 83 % d.Th. resultiert, bezogen auf den im Unterschuss eingesetzten Carbonsäureester. Dieses Verfahren hat jedoch den Nachteil, dass ein 13-14facher Überschuss an Acetonitril angewendet wird, was aus gewerbe-hygienischer Sicht nicht erwünscht ist.

Die Umsetzung von Carbonsäureestern mit Acetonitril unter der Katalyse von Natriumethylat im stöchiometrischen Verhältnis ist zwar in J. Am. Chem. Soc. 56, 1172 (1934) beschrieben, ergibt jedoch mit 44 % d.Th. nur mäßige Ausbeuten.

Es bestand daher die Aufgabe, ein Verfahren zu finden, dass einen Überschuss an Acetonitril vermeidet und gute Ausbeuten ermöglicht.

Die Vermeidung von einem Überschuss an Acetonitril, das bekanntlich als polaraprotisches Lösungsmittel sehr gute Lösungseigenschaften besitzt, führt zu verfahrenstechnischen Problemen und zu einer Verschlechterung der Ausbeute der gewünschten β-Ketonitrile. So kann beispielsweise die Vermeidung eines Acetonitril-Überschusses zu einer schwer rührbaren Reaktionsmischung (Verbreiung) führen. Diese Verbreiung ist insbesondere problematisch, wenn vor der Hydrolyse des die β-Ketonitrile enthaltenden Reaktionsgemisches auf niedrigere Temperaturen wie z.B. Raumtemperatur abgekühlt wird, um die Ausbeute der hydrolyse-empfindlichen β-Ketonitrile nicht zu mindern.

Überraschenderweise fanden wir, dass durch die Verwendung eines Überschusses an Carbonsäureester, bezogen auf die Menge an Acetonitril, die dieser Erfindung zu Grunde liegende Aufgabe gelöst werden kann.

Gegenstand der Erfindung ist somit ein Verfahren zur Herstellung von β-Ketonitrilen der Formel (I) 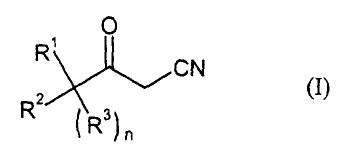 worin
- n =: 0 oder 1 sein kann und für den Fall n = 1
- R¹ =: H oder Methyl
- R² und R³: unabhängig voneinander Methyl oder Ethyl oder
- R² und R³: zusammen einen gegebenenfalls substituierten 3 bis 6 gliedrigen Ring
oder im Fall von n = 0
R¹ und R² zusammen einen gegebenenfalls substituierten 3 bis 6 gliedrigen Ring
bedeuten können,
durch Umsetzung von Acetonitril mit Carbonsäureestern der Formel (II) 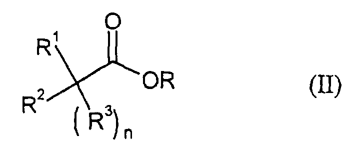 worin
- n: sowie
- R¹ bis R³ die: oben angegebene Bedeutung haben und
- R: ein C₁ bis C₄-Alkylrest sein kann,
in Gegenwart eines Alkalialkoholats, dadurch gekennzeichnet, dass das molare Verhältnis von Carbonsäureester (II) zu Acetonitril im Bereich von 2:1 bis 10:1 liegt.

Für den Fall, dass R² und R³ zusammen einen 3 bis 6 gliedrigen Ring bedeuten, sind Cyclopropyl, Cyclopentyl und Cyclohexyl bevorzugt. Die Ringe können zusätzlich eine Doppelbindung oder gegebenenfalls zwei Doppelbindungen enthalten. Gegebenenfalls können die Ringe auch Heteroatome wie N, S, oder O enthalten.

Für den Fall, dass R¹ und R² zusammen einen gegebenenfalls substituierten 3 bis 6 gliedrigen Ring bedeuten, sind aromatische Reste, gegebenenfalls mit ein oder zwei Heteroatomen im Ring, bevorzugt. Besonders bevorzugt sind Phenylreste. Bei substituierten Phenylresten ist als Substituent ein Niederalkylrest oder ein Niederalkoxyrest bevorzugt, insbesondere die Substituenten Methoxy oder Methyl. Bevorzugt ist ein Substituent in para-Position.

Bevorzugt ist die Herstellung von 4-Methyl-3-oxo-valeronitril, 4,4-Dimethyl-3-oxovaleronitril, 3-Cyclopropyl-3-oxo-propionitril, 3-Cyclopentyl-3-oxo-propionitril, 3-Cyclohexyl-3-oxo-propionitril, 3-Phenyl-3-oxo-propionitril, 3-(p-Methoxyphenyl)-3-oxo-propionitril und 3-(p-Methylphenyl)-3-oxo-propionitril, besonders bevorzugt sind 4-Methyl-3-oxo-valeronitril, 4,4-Dimethyl-3-oxo-valeronitril, 3-Cyclopropyl-3-oxo-propionitril und 3-Phenyl-3-oxo-propionitril.

Bevorzugt ist R = Methyl (Me) oder Ethyl (Et), besonders bevorzugt ist R = Methyl.

Die Carbonsäureester werden im allgemeinen im 2 bis 10-fachen molaren, vorzugsweise im 3 bis 5-fachen molaren Überschuss bezogen auf Acetonitril eingesetzt.

Das molare Verhältnis von Alkalialkoholat zu Acetonitril liegt bevorzugt im Bereich von 0,8 bis 1,4:1, besonders bevorzugt im Bereich von 1,0 bis 1,2:1.

Bevorzugte Alkalialkoholate sind KOMe, NaOMe, KOEt und NaOEt. Diese Alkalialkoholate können auch als Gemische eingesetzt werden. Besonders bevorzugt ist Natriummethylat.

Die Reaktion kann in Gegenwart eines inerten Lösungsmittels durchgeführt werden, bevorzugt ist die lösungsmittelfreie Reaktionsführung.

Das Verfahren wird bei Temperaturen durchgeführt, die im Bereich der Siedepunkte der aus der Reaktion entstehenden Alkohole liegen, typischerweise zwischen 60 bis 120°C oder auch bei Temperaturen, die sich aufgrund von azeotropen Gemischen zwischen diesen Alkoholen und Acetonitril (z.B. Methanol/Acetonitril) bilden.

Das Verfahren wird vorteilhaft unter Abdestillieren des während der Reaktion entstehenden Alkohols ROH ausgeführt. Die Reaktion ist in der Regel nach 4 bis 5 Stunden beendet.

Die Hydrolyse ist auch bei deutlich höheren Temperaturen als Raumtemperatur möglich, ohne merkliche Ausbeuteverluste hinnehmen zu müssen. So lassen sich bei dieser speziellen Ausführungsform Ausbeuten von 85 % d.Th. erzielen, bezogen auf den Umsatz des im Unterschuss eingesetzten Acetonitrils.

Die Aufarbeitung wird also bevorzugt durch Hydrolyse in der Art durchgeführt, dass man die Reaktionsmischung mit einer Temperatur von 60 bis 90°C, vorzugsweise 75 bis 80°C, unter Rühren zügig in Wasser einbringt. Die Temperatur des Wassers liegt hierbei typischerweise im Bereich von 0 bis 30°C.

Das Produkt liegt nach der Hydrolyse als Alkali-Enolat in der wässrigen Phase vor und wird durch Ansäuern mit einer Mineralsäure (z.B. Salz- oder Schwefelsäure) als Keton freigesetzt und anschließend mit einem nicht Wasser-mischbaren organischen Lösungsmittel extrahiert. Die Reinigung kann gegebenenfalls durch Destillation oder Kristallisation erfolgen.

Der überschüssige Carbonsäureester trennt sich nach der Hydrolyse als obere organische Phase ab und kann nach Abtrennung und Trocknung wieder in die Reaktion eingesetzt werden. Diese Trocknung wird vorteilhaft durch Zusatz eines leicht-flüchtigen Kohlenwasserstoffs (z.B. Heptan, Cyclohexan), der mit Wasser ein Azeotrop bildet, durchgeführt, in dem man mit Hilfe des azeotropen Gemisches den Carbonsäureester vom Wasser destillativ befreit.

Die Aufarbeitung kann auch derart erfolgen, dass nach Hydrolyse angesäuert wird und sich der überschüssige Carbonsäureester zusammen mit dem Produkt als organische Phase absetzen. Die Verwendung eines zusätzlichen Lösungsmittels entfällt dabei.

### Beispiel 1:

### 4,4-Dimethyl-3-oxo-valeronitril

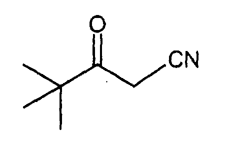

812 g (7,0 mol) Pivalinsäuremethylester, 82 g (2,0 mol) Acetonitril und 119 g (2,2 mol) Natriummethylat wurden in einen 2-1 Doppelmantel mit Bodenablassventil eingebracht und 1,5 h unter Rühren auf 88-90°C erhitzt. Danach wurde 3 h lang ca. 160 g eines Gemisches aus Methanol und Acetonitril, das etwa 13 Gew.-% Acetonitril enthielt, bei einer Kopftemperatur von 70 bis 75°C abdestilliert. Nach Absenkung der Temperatur des Reaktionsgemisches auf 80°C, wurde dieses in 500 g Wasser (Wassertemperatur 20°C) unter Rühren eingebracht. Nach der Phasentrennung wurden 441 g Pivalinsäuremethylester erhalten, die wässrige Phase mit 220 g halbkonzentrierter Schwefelsäure auf pH 5 eingestellt und das Produkt mit 200 g Xylol extrahiert. Nach Aufreinigung durch Destillation wurden 160 g 4,4-Dimethyl-3-oxo-valeronitril erhalten, was einer Ausbeute von 86 % d. Th. entspricht, bezogen auf den Umsatz Acetonitril.

Alternativ dazu kann die Aufarbeitung so erfolgen, dass nach Hydrolyse ohne Zusatz von einem Lösungsmittel (wie z.B. Xylol) auf pH 6 bis 7 angesäuert wird und somit das Produkt und der wiedergewonnene Pivalinsäuremethylester zusammen sich als organische Phase abtrennen. Eine anschließende Destillation führt zum gleichen Ausbeute-Ergebnis.

### Beispiel 2:

### 4-Methyl-3-oxo-valeronitril

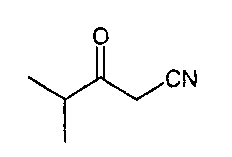

Es wird in gleicher Weise verfahren wie in Beispiel 1 unter Einsatz entsprechender Mengen von Isobuttersäureethylester, Acetonitril und Natriumethylat. Die Ausbeute an 4-Methyl-3-oxo-valeronitril lag bei 84 % d.Th., bezogen auf den Umsatz Acetonitril.

### Beispiel 3:

### 3 -Cyclopropyl-3 -oxo-propionitril

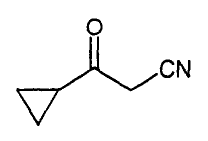

Es wird in gleicher Weise verfahren wie in Beispiel 1 unter Einsatz entsprechender Mengen von Cyclopropancarbonsäuremethylester, Acetonitril und Natriumethylat. Die Ausbeute an 3-Cyclopropyl-3-oxo-propionitril lag bei 82 % d.Th., bezogen auf den Umsatz Acetonitril.

### Beispiel 4:

### 3-Phenyl-3-oxo-propionitril

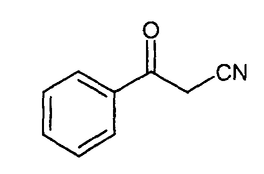

Es wird in gleicher Weise verfahren wie in Beispiel 1 unter Einsatz entsprechender Mengen von Benzoesäuremethylester, Acetonitril und Natriumethylat. Die Ausbeute an 3-Phenyl-3-oxo-propionitril lag bei 85 % d.Th., bezogen auf den Umsatz Acetonitril.

## Patentansprüche

1. Verfahren zur Herstellung von β-Ketonitrilen der Formel (I) 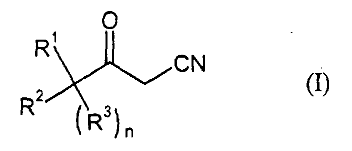 worin
n = 0 oder 1 sein kann und für den Fall n = 1
R¹ = H oder Methyl
R² und R³ unabhängig voneinander Methyl oder Ethyl oder
R² und R³ zusammen einen gegebenenfalls substituierten 3 bis 6 gliedrigen Ring
oder im Fall von n = 0
R¹ und R² zusammen einen gegebenenfalls substituierten 3 bis 6 gliedrigen Ring
bedeuten können,
durch Umsetzung von Acetonitril mit Carbonsäureestern der Formel (II) 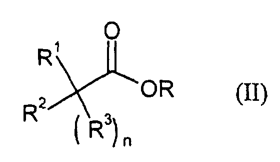 worin
n sowie
R¹ bis R³ die oben angegebene Bedeutung haben und
R ein C₁ bis C₄-Alkylrest sein kann,
in Gegenwart eines Alkalialkoholats, **dadurch gekennzeichnet, dass** das molare Verhältnis von Carbonsäureester (II) zu Acetonitril im Bereich von 2:1 bis 10:1 liegt.

2. Verfahren nach Anspruch 1, **dadurch gekennzeichnet, dass** als Alkalialkoholat Natriummethylat, Natriumethylat, Kaliummethylat oder Kaliumethylat oder deren Gemisch eingesetzt wird.

3. Verfahren nach mindestens einem der Ansprüche 1 oder 2, **dadurch gekennzeichnet, dass** bei der Reaktion von Acetonitril und Carbonsäureester (II) kein Lösungsmittel zugesetzt wird.

4. Verfahren nach mindestens einem der Ansprüche 1 bis 3, **dadurch gekennzeichnet, dass** der entstehende Alkohol aus dem Reaktionsgemisch abdestilliert wird.

5. Verfahren nach mindestens einem der Ansprüche 1 bis 4, **dadurch gekennzeichnet, dass** das Reaktionsgemisch bei einer Temperatur von 60-90°C hydrolysiert wird.

## Claims

1. Process for the production of β-ketonitriles of formula (I) 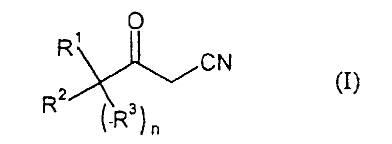 wherein
n can be 0 or 1 and, where n = 1,
R¹ is H or methyl,
R² and R³ independently of one another can denote methyl or ethyl or
R² and R³ together can denote an optionally substituted, 3- to 6-membered ring
or, where n = 0,
R¹ and R² together can denote an optionally substituted, 3- to 6-membered ring,
by reacting acetonitrile with carboxylic esters of the formula (II) 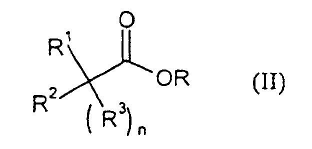 wherein
n as well as
R¹ to R³ have the meaning given above and
R can be a C₁ to C₄ alkyl radical,
in the presence of an alkali alcoholate, **characterised in that** the molar ratio of carboxylic ester (II) to acetonitrile is in the range of 2:1 to 10:1.

2. Process according to claim 1, **characterised in that** sodium methylate, sodium ethylate, potassium methylate or potassium ethylate or a mixture thereof is used as the alkali alcoholate.

3. Process according to at least one of claims 1 or 2, **characterised in that** no solvent is added during the reaction of acetonitrile and carboxylic ester (II).

4. Process according to at least one of claims 1 to 3, **characterised in that** the resulting alcohol is distilled out of the reaction mixture.

5. Process according to at least one of claims 1 to 4, **characterised in that** the reaction mixture is hydrolysed at a temperature of 60-90°C.

## Revendications

1. Procédé pour la préparation de β-cétonitriles de formule (I) 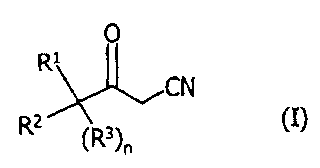 dans laquelle
n = 0 ou 1 et, lorsque n =1
R¹ = H ou méthyle,
R² et R³ représentent chacun, indépendamment l'un de l'autre, un groupe méthyle ou éthyle ou bien
R² et R³ forment ensemble un cycle de 3 à 6 chaînons éventuellement substitué,
ou bien, lorsque n = 0,
R¹ et R² forment ensemble un cycle de 3 à 6 chaînons éventuellement substitué,
par réaction de l'acétonitrile avec des esters d'acides carboxyliques de forrnule (II) 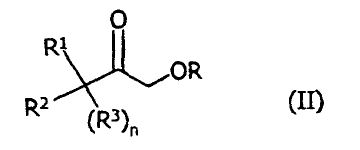 dans laquelle
n et
R¹ à R³ ont les significations indiquées ci-dessus et
R représente un groupe alkyle en C₁-C₄,
en présence d'un alcoolate alcalin, ce procédé **se caractérisant en ce que** le rapport molaire ester d'acide carboxylique (II)/acétonitrile se situe dans l'intervalle de 2:1 à 10:1.

2. Procédé selon la revendication 1, **caractérisé en ce que** l'alcoolate alcalin utilisé est le méthylate de sodium, l'éthylate de sodium, le méthylate de potassium ou l'éthylate de potassium ou un mélange de ces alcoolates.

3. Procédé selon au moins une des revendications 1 ou 2 **caractérisé en ce que**, à la réaction entre l'acétonitrile et l'ester d'acide carboxylique (II), on n'utilise pas de solvant.

4. Procédé selon au moins une des revendications 1 à 3 **caractérisé en ce que** l'alcool formé est distillé du mélange de réaction.

5. Procédé selon au moins une des revendications 1 à 4 **caractérisé en ce que** l'on hydrolyse le mélange de réaction à une température de 60 à 90°C.
